# EUROPÄISCHE PATENTSCHRIFT

(11) **EP 2 403 361 B1**
(45) Veröffentlichungstag und Bekanntmachung des Hinweises auf die Patenterteilung: **26.12.2012**
(21) Anmeldenummer: 10707006.2
(22) Anmeldetag: 03.03.2010
(51) Int. Cl.: A23L 1/275, F28D 3/00, C07C 403/24

(54) **VERFAHREN ZUR HERSTELLUNG VON CAROTINOID-LÖSUNGEN**
METHOD FOR PRODUCING CAROTENOID SOLUTIONS
PROCÉDÉ DE FABRICATION DE SOLUTIONS DE CAROTÉNOÏDE

(30) Priorität: 04.03.2009 EP 09154304
(43) Veröffentlichungstag der Anmeldung: 11.01.2012
(73) Patentinhaber: BASF SE, 67056 Ludwigshafen (DE)
(72) Erfinder: ENGEL, Robert, 67346 Speyer (DE); RUMPF, Bernd, 68766 Hockenheim (DE)
(86) Internationale Anmeldenummer: PCT/EP2010/052692
(87) Internationale Veröffentlichungsnummer: WO 2010/100188

(56) Entgegenhaltungen:
- WO-A1-2006/125591
- US-A1- 2001 008 644

## Beschreibung

Die vorliegende Erfindung betrifft ein Verfahren zur Herstellung von Carotinoid-Lösungen in einem für Nahrungsmittel geeigneten Öl. Insbesondere betrifft die Erfindung ein Verfahren zum Auflösen eines Carotinoids, insbesondere Astaxanthin, in einem für Nahrungsmittel geeigneten Öl durch Erhitzen einer Suspension des Carotinoids, speziell des Astaxanthins, in dem Öl und anschließendes Abschrecken (Quenchen) der erhaltenen Lösung.

Carotinoide werden in großem Umfang zu pharmazeutischen oder kosmetischen Zwecken sowie in der Nahrungsmittelindustrie eingesetzt. In der Nahrungsmittelindustrie werden Carotinoide als farbgebende Additive und essentielle Nahrungsergänzungsmittel verwendet. Ein wesentliches Problem bei der Verabreichung von Carotinoiden ist deren geringe Wasserlöslichkeit und die damit einhergehende schlechte Bioverfügbarkeit. Dieses Problem ist besonders bei Astaxanthin ausgeprägt. Aus diesem Grund können Carotinoide und insbesondere Astaxanthin und seine Derivate nicht als solche eingesetzt werden, sondern müssen in eine Formulierung überführt werden, die eine hinreichende Bioverfügbarkeit dieser Substanzen gewährleistet. Aufgrund der chemischen Instabilität von Carotinoiden - die Verbindungen sind oxidationslabil und neigen zudem zur Bildung von inaktiven Konfigurationsisomeren durch cis-trans-Isomerisierung der exocyclischen Doppelbindungen - stellt die Formulierung dieser Verbindungen eine besondere Herausforderung dar. Hierbei sind flüssige Formulierungen der Carotinoide, speziell des Astaxanthins, in essbaren, d. h. für Nahrungsmittel geeigneten, Ölen von besonderem Interesse.

Die WO2006/125591 beschreibt die Herstellung von Carotinoid-Lösungen in für Nahrungsmittel geeigneten Ölen, bei dem man zunächst eine Suspension des Carotinoids in der Ölphase bereitstellt, die Suspension für eine kurze Zeit durch kontinuierliches Vermischen mit einem heißen Öl erhitzt, um das Carotinoid in Lösung zu bringen und die so erhaltene heiße Carotinoid-Lösung abschreckt, indem man sie mit einem kalten, für Nahrungsmittel geeigneten Öl kontinuierlich vermischt. Dieses Verfahren ist mit einer Reihe von Nachteilen verbunden. Zum einen führt das Abschrecken mit dem kalten Öl zu einer starken Verdünnung der erhaltenen Carotinoid-Lösung, so dass man in der heißen Carotinoid-Lösung eine vergleichsweise hohe Carotinoid-Konzentration einstellen muss. Aufgrund der schlechten Löslichkeit und der geringen Lösungsgeschwindigkeit von Carotinoiden in solchen Ölen müssen vergleichsweise hohe Temperaturen angewendet werden und/oder die Teilchengrößen des Carotinoids in der Suspension müssen sehr gering sein. So sind in der Regel bei Astaxanthin Temperaturen oberhalb 150 °C erforderlich, um bei vertretbaren Partikelgrößen (D_{4.3} > 2 µm) eine Konzentration von 1500 ppm in der heißen Lösung zu erreichen. Bei diesen Temperaturen tritt jedoch bereits ein merklicher Abbau des Astaxanthins durch Bildung der unerwünschten cis-Isomeren auf. Zudem besteht bei nicht-raffinierten Ölen die Gefahr, dass sich in den Apparaturen Ablagerungen durch Zersetzungsreaktionen im Öl bilden.

Der vorliegenden Erfindung liegt somit die Aufgabe zu Grunde, ein effizienteres Verfahren zur Herstellung von Carotinoid-Lösungen in für Nahrungsmittel geeigneten Ölen bereitzustellen, das insbesondere die Nachteile des Standes der Technik behebt. Diese Aufgabe wird durch das im Folgenden beschriebene Verfahren gelöst.

Die Erfindung betrifft somit ein Verfahren zur Herstellung von Carotinoid-Lösungen in einem für Nahrungsmittel geeigneten Öl, umfassend:
i. Bereitstellung einer Suspension des Carotinoids in einem für Nahrungsmittel geeigneten Öl,
ii. kontinuierliches Erhitzen eines Stroms der Carotinoid-Suspension, wobei man eine heiße Carotinoid-Lösung erhält, und
iii. kontinuierliches Abschrecken der heißen Carotinoid-Lösung,
wobei man zum kontinuierlichen Erhitzen die Carotinoid-Suspension als dünnen Flüssigkeitsfilm über eine beheizte Wärmetauscherfläche führt.

Das erfindungsgemäße Verfahren ist mit einer Reihe von Vorteilen verbunden. Die erfindungsgemäße Durchführung des Erhitzens erlaubt ein besonders rasches Erhitzen der Carotinoid-Suspension auf eine zum Lösen des Carotinoids erforderliche Temperatur, da durch die erfindungsgemäße Vorgehensweise hohe Wärmeübergangswerte erreicht werden. Dies führt nicht nur zu einer Verkürzung der für das Lösen erforderlichen Zeit, sondern erlaubt auch die Herstellung konzentrierter Lösungen, ohne dass hohe Temperaturen über einen längeren Zeitraum angewendet werden müssen. Das erfindungsgemäße Erhitzen führt zudem zu einem besonders gleichmäßigen Temperatureintrag in die Carotinoid-Suspension, und Temperaturspitzen, welche eine Zersetzung oder Isomerisierung des Carotinoids bewirken würden, werden effizient vermieden. Zudem kann man auf diese Weise ein Fouling des Öls verringern oder gar vermeiden. Durch das Abschrecken wiederum wird ein Auskristallisieren des Carotinoids weitgehend oder vollständig vermieden. Hierbei ist zu beachten, dass bereits geringe Mengen an nicht gelöstem Carotinoid in der Produktlösung stören und sich auch nur äußerst langsam, wenn überhaupt, wieder lösen. Zudem wird durch das rasche Abkühlen effizient die Zersetzung oder Isomerisierung des Carotinoids verringert.

Das erfindungsgemäße Verfahren wird nunmehr im Folgenden ausführlich erläutert. Bevorzugte Ausführungsformen des erfindungsgemäßen Verfahrens sind im Folgenden und in den Unteransprüchen angegeben.

Unter einem für Nahrungsmittel geeigneten Öl versteht man im Sinne der Erfindung ein für die Tier- und/oder Humanernährung zugelassenes Öl. Dieses Öl wird im Folgenden auch als essbares Öl bezeichnet. Das essbare Öl kann synthetischer, mineralischer, pflanzlicher oder tierischer Herkunft sein. Beispiele sind Pflanzenöle wie Sojabohnenöl, Palmöl, Palmkernöl, Sonnenblumenöl, Maiskeimöl, Leinsamenöl, Baumwollsaatöl, Tocopherole, Rapsöl, Distelöl, Weizenkeimöl, Reisöl, Kokosöl, Mandelöl, Aprikosenkemöl, Avocadoöl, Jojobaöl, Haselnussöl, Walnussöl, Erdnussöl, Pistazienöl, Triglyceride mittelkettiger (= C₈-C₁₀) Fettsäuren pflanzlichen Ursprungs (sog. MCT-Öle) und PUFA-Öle (PUFA = mehrfach ungesättigte Fettsäuren (polyunsaturated fatty acids) wie Eikosapentaensäure (EPA), Docosahexaensäure (DHA) und α-Linolensäure), weiterhin semisynthetische Triglyceride, z. B. Caprylsäure/Caprinsäure-Triglyceride wie die Miglyol-Typen, weiterhin Oleostearin, Paraffinöl, Glycerylstearat, Isopropylmyristat, Diisopropyladipat, 2-Ethylhexansäureacetylstearylester, flüssige hydrierte Polyisobutene, Squalan, Squalen, weiterhin tierische Öle und Fette wie Schmalz, Talg, Fischöle einschließlich Makrelen-, Sprotten-, Thunfisch-, Heilbutt-, Kabeljau- und Lachsöl sowie kommerzielle Fischölblends wie Südamerikanisches Fischöl, Skandinavisches Fischöl und Menhaden Fischöl, sowie weiterhin Lanolin. Geeignet sind selbstredend auch Mischungen dieser Öle.

Bevorzugt sind insbesondere Pflanzenöle wie Sojabohnenöl, Palmöl, Palmkernöl, Sonnenblumenöl, Distelöl, Maiskeimöl, Olivenöl, Leinsamenöl, Rapsöl, Reisöl, Kokosöl, Erdnussöl, PUFA-Öle, MCT-Öle, weiterhin Fischöle, Fischölblends sowie Mischungen dieser Öle, insbesondere Maisöl, Sonnenblumenöl, Sojabohnenöl, Fischöl, Palmkernöl und MCT-Öle.

In einer besonders bevorzugten Ausführungsform der Erfindung umfasst das zur Herstellung der heißen Carotinoid-Lösungen und Carotinoid-Suspensionen verwendete Öl zu wenigstens 50 Gew.-%, insbesondere zu wenigstens 80 Gew.-%, bezogen auf die Gesamtmenge der in den Schritten i. und gegebenenfalls ii. eingesetzten Öle, wenigstens ein Öl mit einem hohen Anteil, d. h. wenigstens 60 Gew.-% an C₈- bis C₁₄-Fettsäuren, insbesondere C₈- bis C₁₀-Fettsäuren, insbesondere wenigstens ein unter Palmkernöl und MCT-Ölen ausgewähltes essbares Öl.

Bei den verwendeten Ölen kann es sich um Raffinatöle oder Rohöle handeln, die noch ursprungsspezifische Verunreinigungen wie Proteine, Phosphat, (Erd-)AlkalimetallSalze und dergleichen in üblichen Mengen enthalten. Die im erfindungsgemäßen Verfahren eingesetzten Öle können auch geringe Mengen an Wasser, vorzugsweise jedoch nicht mehr als 10 Gew.-%, z. B. 0,1 bis 10 Gew.-%, häufig 0,5 bis 8 Gew.-%, bezogen auf das Öl, enthalten. In einer speziellen Ausführungsform der Erfindung enthält das essbare Öl nicht mehr als 0,5 Gew.-%, bezogen auf die Gesamtmenge des eingesetzten essbaren Öls, an Wasser.

Erfindungsgemäß geeignete Carotinoide sind alle bekannten, aus natürlichen Quellen oder synthetisch zugänglichen Carotinoide und Carotinoid-Derivate. Beispiele hierfür sind Carotene wie ß-Carotin und Lycopin, sowie Xanthophylle wie Lutein, Astaxanthin, Adonirubin, Adonixanthin, Zeaxanthin, Cryptoxanthin, Citranaxanthin, Canthaxanthin, Echinenon, Bixin, ß-Apo-4-carotinal, ß-Apo-8-carotinal, ß-Apo-4-carotinsäureester, einzeln oder als Mischung. Die erfindungsgemäßen Vorteile kommen insbesondere bei Xanthophyllen und speziell bei Astaxanthin und Canthaxanthin zum Tragen und insbesondere bei Astaxanthin. Dementsprechend betrifft eine besonders bevorzugte Ausführungsform der Erfindung ein Verfahren, bei dem als Carotinoid Astaxanthin verwendet wird.

Sofern es sich bei dem Carotinoid um Astaxanthin handelt, wird dieses in Schritt i. üblicherweise in einer Reinheit, bezogen auf das all-trans-Isomer von wenigstens 70 Gew.-%, häufig wenigstens 80 Gew.-%, vorzugsweise wenigstens 90 Gew.-% und insbesondere wenigstens 95 Gew.-% eingesetzt. Neben dem all-trans-Isomeren kann das Astaxanthin auch Anteile an cis-Isomeren des Astaxanthins sowie von Astaxanthin verschiedene Carotinoide enthalten. Der Anteil an von Astaxanthin verschiedenen Carotinoiden wird in der Regel 30 Gew.-%, häufig 20 Gew.-%, insbesondere 10 Gew.-% und besonders bevorzugt 5 Gew.-%, bezogen auf die Gesamtmenge des eingesetzten Carotinoids nicht überschreiten.

Insbesondere verwendet man Astaxanthin, das die an ein für Nahrungsmittel zugelassenes Astaxanthin gestellten Anforderungen erfüllt, d. h. das weniger als 4 Gew.-% Carotinoide, die von Astaxanthin verschieden sind, enthält, und das einen Schwermetallgehalt von höchstens 10 ppm aufweist und das zu wenigstens 70 Gew.-%, häufig wenigstens 80 Gew.-%, vorzugsweise wenigstens 90 Gew.-% aus dem all-trans-Isomeren besteht.

Die Bereitstellung einer Suspension des Carotinoids in dem für Nahrungsmittel geeigneten Öl erfolgt nach üblichen Verfahren zur Bereitstellung solcher Suspensionen, wie sie im Stand der Technik beschrieben sind. Hierzu kommen grundsätzlich Verfahren in Betracht, bei denen ein durch Fällung oder in sonstiger Weise hergestelltes Pulver des Carotinoids in Öl suspendiert wird, sowie Verfahren, bei denen ein festes Carotinoid in dem Öl durch Vermahlen auf die gewünschte Teilchengröße gebracht wird.

Die Carotinoid-Suspension kann kontinuierlich oder diskontinuierlich hergestellt werden.

Insbesondere erfolgt die Bereitstellung der Carotinoid-Suspension in Schritt i. des erfindungsgemäßen Verfahrens durch Vermahlen des Carotinoids, welches vorzugsweise die oben genannte Reinheit aufweist, insbesondere von Astaxanthin, in dem für Nahrungsmittel geeigneten Öl. Hierzu wird man in der Regel das Carotinoid zunächst in dem Öl suspendieren, wobei man eine grobteilige Carotinoid-Suspension erhält, und anschließend die Carotinoid-Partikel in einer geeignete Vorrichtung zum Vermahlen auf die gewünschte Teilchengröße zerkleinern. Als Vorrichtungen zum Vermahlen können übliche, dem Fachmann bekannte Vorrichtungen, beispielsweise Kugelmühlen, Perlmühlen oder Kolloidmühlen, eingesetzt werden.

Das Vermahlen wird in der Regel so durchgeführt, dass die Partikel des Carotinoids in der Suspension einen volumenmittleren Teilchendurchmesser im Bereich von 0,5 bis 50 µm, insbesondere im Bereich von 0,6 bis 30 µm und speziell im Bereich von 0,7 bis 20 µm aufweisen. Ganz speziell liegt der volumenmittlere Teilchendurchmesser im Bereich von 0,8 bis 15 µm. Unter dem volumenmittleren Teilchendurchmesser versteht man den mittels Fraunhofer-Beugung an einer verdünnten 0,01 bis 0,1 gew.-%igen Suspension des Carotinoids in dem essbaren Öl bestimmten volumenmittleren Teilchendurchmesser (D_{4,3}-Wert).

Das Vermahlen erfolgt typischerweise bei Temperaturen unterhalb 100 °C, häufig im Bereich von 20 bis 90 °C und insbesondere im Bereich von 30 bis 70 °C. Das Vermahlen wird solange durchgeführt, bis die gewünschte Teilchengröße erreicht ist.

Die Konzentration an Carotinoid in der öligen Suspension wird in der Regel so gewählt, dass sie im Bereich von 0,1 bis 30 Gew.-%, insbesondere im Bereich von 0,5 bis 20 Gew.-% liegt. Dementsprechend wird man zum Vermahlen entsprechende Mengenverhältnisse an Öl und Carotinoid einsetzen.

Gegebenenfalls können im erfindungsgemäßen Verfahren übliche Mittel zur Stabilisierung von Carotinoid-Lösungen oder -Suspensionen wie lipophile Dispergiermittel, Antioxidantien (Oxidationsstabilisatoren) und dergleichen eingesetzt werden. Diese Mittel können bei der Bereitstellung der Suspension, beim Erhitzen der Suspension, z. B. über einen heißen Ölstrom, und/oder beim Abschrecken der der heißen Öllösung über die kühlere Carotinoid-Lösung zugeführt werden.

Beispiele für Antioxidantien sind Tocopherole wie α-Tocopherol, α-Tocopherolpalmitat, α-Tocopherolacetat, tert.Butylhydroxytoluol, tert.-Butylhydrochinon, tert.-Butylhydroxyanisol, Ascorbinsäure, ihre Salze und Ester, z. B. Natriumascorbat, Calciumascorbat, Ascorbylphosphatester, und Ethoxyquin. Die Antioxidantien werden, sofern erwünscht, typischerweise in Mengen von 0,001 bis 20 Gewichtsteile, bezogen auf 1 Gewichtsteil des Carotinoids eingesetzt. Im Falle von Tocopherolen kann die eingesetzte Menge an Stabilisator auch höher sein.

Typische lipophile Dispergiermittel sind Ascorbylpalmitat, Polyglycerin-Fettsäureester wie Polyglycerin-3-polyrizinoleat (PGPR90), Sorbitanfettsäureester wie Sorbitanmonostearat (SPAN60), PEG(20)-Sorbitolmonooleat, Propylenglycol-Fettsäureester sowie Phospholipide wie Lecithin. Lipophile Dispergiermittel werden, sofern erwünscht, üblicherweise in Mengen von 0,1 bis 10 Gew.-Teilen, bezogen auf 1 Gew.-Teil des Carotinoids, eingesetzt. In einer bevorzugten Ausführungsform der Erfindung wird kein lipophiles Dispergiermittel eingesetzt.

Weiterhin können der Suspension Zuschlagsstoffe zugegeben werden, z. B. Basen wie Ammoniak, basische Aminosäuren oder basische Adsorbentien, um die Acidität des verwendeten Öls zu erniedrigen und damit eine weitere Erniedrigung des Isomerisierungsgrades zu erreichen, sowie inerte Zusätze wie unlösliche Sulfate der Erdalkalimetalle, die als harte Festkörper die Mahlung beschleunigen und in der Suspension verbleiben können, weil sie physiologisch unbedenklich sind. Insbesondere geeignet sind Alkalimetall- und Erdalkalimetallcarbonate, da sie als Festkörper die Mahlung beschleunigen, in der Suspension verbleiben können, weil sie physiologisch unbedenklich sind, und zudem zur Verminderung der Acidität der verwendeten Öle beitragen. In einer besonders bevorzugten Ausführungsform der Erfindung umfasst das zur Herstellung der Carotinoid-Suspensionen verwendete Öl zu wenigstens 50 Gew.-%, insbesondere zu wenigstens 80 Gew.-%, wenigstens ein Öl mit einem hohen Anteil, d. h. wenigstens 60 Gew.-% an C₈- bis C₁₄-Fettsäuren, insbesondere C₈- bis C₁₀-Fettsäuren, insbesondere wenigstens ein unter Palmkernöl und MCT-Ölen ausgewähltes essbares Öl.

Das zur Herstellung der Suspension eingesetzte essbare Öl ist üblicherweise unter den vorgenannten essbaren Ölen, insbesondere unter den als bevorzugt oder besonders bevorzugt genannten essbaren Ölen ausgewählt. Zur Herstellung der Suspension werden essbare Öle bevorzugt, die bei 30 °C flüssig sind.

Das zur Herstellung der Suspension eingesetzte Öl kann geringe Mengen an Antioxidantien enthalten. Die Antioxidantien können auch vor, während oder nach dem Mahlen der Suspension zugesetzt werden. Daneben kann das eingesetzte Öl auch geringe Mengen an Wasser enthalten, vorzugsweise jedoch nicht mehr als 10 Gew.-%, z. B. 0,1 bis 10 Gew.-%, häufig 0,5 bis 8 Gew.-%, bezogen auf das zur Herstellung der Suspension eingesetzte Öl. In einer speziellen Ausführungsform der Erfindung enthält das zur Herstellung der Suspension eingesetzte essbare Öl nicht mehr als 0,5 Gew.-% an Wasser.

In Schritt ii. des erfindungsgemäßen Verfahrens wird die Carotinoid-Suspension kontinuierlich erhitzt. Hierzu wird der Strom der Carotinoid-Suspension kontinuierlich als dünner Flüssigkeitsfilm über eine beheizte Wärmetauscherfläche geführt. Hierbei kann man die in Schritt i. hergestellte Suspension als solche oder nach Verdünnung mit einem für Nahrungsmittel geeigneten Öl als dünnen Flüssigkeitsfilm über die beheizte Wärmetauscherfläche führen.

Vorzugsweise wird die Konzentration an Carotinoid in dem Strom der Suspension, der als Flüssigkeitsfilm über die Wärmetauscherfläche geführt wird, im Bereich von 0,5 bis 50 g/kg und insbesondere im Bereich von 1 bis 20 g/kg liegen. Diese Konzentration kann beim Herstellen der Suspension direkt eingestellt werden. Bei geringerer Konzentration wird man vorzugsweise zunächst eine konzentriertere Suspension herstellen und diese anschließend mit einem für Nahrungsmittel geeigneten Öl auf die zum Erhitzen bevorzugte Konzentration verdünnen.

Das Öl, welches gegebenenfalls zum Verdünnen der Suspension eingesetzt wird, ist ein für Nahrungsmittel geeignetes Öl, das üblicherweise unter den vorgenannten, für Nahrungsmittel geeigneten Ölen, insbesondere unter den als bevorzugt oder besonders bevorzugt genannten essbaren Ölen ausgewählt ist. Das zugeführte Öl kann geringe Mengen an Antioxidantien enthalten. In einer besonders bevorzugten Ausführungsform der Erfindung umfasst das in Schritt ii. zugeführte Öl zu wenigstens 50 Gew.-%, insbesondere zu wenigstens 80 Gew.-%, wenigstens ein Öl mit einem hohen Anteil, d. h. wenigstens 60 Gew.-% an C₈- bis C₁₄-Fettsäuren, insbesondere C₈-bis C₁₀-Fettsäuren, insbesondere wenigstens ein unter Palmkernöl und MCT-Ölen ausgewähltes essbares Öl.

Der zu erhitzende Strom aus Carotinoid-Suspension, die gegebenenfalls mit einem für Nahrungsmittel geeigneten Öl verdünnt worden ist, wird im Folgenden auch als Ölstrom bezeichnet.

Erfindungsgemäß wird der Ölstrom als dünner Flüssigkeitsfilm über die beheizte Wärmetauscherfläche geführt bzw. geleitet. Hierzu wird ein dünner Flüssigkeitsfilm auf der beheizten Wärmetauscherfläche erzeugt. Durch den Kontakt mit der heißen Wärmetauscherfläche wird der Flüssigkeitsfilm erhitzt, das suspendierte Carotinoid löst sich in dem Öl und es bildet sich ein dünner Flüssigkeitsfilm der heißen Carotinoid-Lösung. Diese wird von der beheizten Wärmetauscherfläche abgeführt/abgeleitet und abgeschreckt, d. h. rasch auf eine niedrige Temperatur gebracht. Die im erfindungsgemäßen Verfahren eingesetzten Vorrichtungen werden im Folgenden auch als Dünnschichtapparaturen bezeichnet.

Die Auftragsmenge an Suspension, d. h. die Beladung der Dünnschichtapparatur (Berieselungsdichte) wird so gewählt, dass sich ein dünner Flüssigkeitsfilm bildet, der in der Regel eine Dicke von 2 mm nicht überschreitet und der typischerweise eine mittlere Dicke im Bereich von 0,02 bis 2 mm und häufig im Bereich von 0,05 bis 1 mm liegt. Die Berieselungsdichte hängt naturgemäß von der Art der Wärmetauscheranordnung ab und liegt typischerweise im Bereich von 0,1 bis 2 m³/(m*h).

Die Dünnschichtapparaturen sind dem Fachmann grundsätzlich aus dem Bereich der Dünnschichtverdampfungstechnik vom Prinzip her bekannt, z. B. aus W. R. A. Vauck und H. A. Müller, Grundoperationen Chemischer Verfahrenstechnik, 10. Auflage, Deutscher Verlag für Grundstoffindustrie, Stuttgart 1994, S. 620-623. Entsprechende Dünnschichtverdampfer können im erfindungsgemäßen Verfahren in analoger Weise eingesetzt werden, wobei die bekannten Dünnschichtverdampfer modifiziert sein können, da beispielsweise in Schritt i. des erfindungsgemäßen Verfahrens Vorrichtungen zur Abtrennung von Brüden nicht benötigt werden. In jedem Fall weisen die im erfindungsgemäßen Verfahren zum Erhitzen eingesetzten Vorrichtungen wenigstens eine beheizbare Wärmetauscherfläche sowie Mittel, die es erlauben, die Carotinoid-Suspension als dünnen Flüssigkeitsfilm über die Wärmetauscheroberfläche zu führen. Hierzu zählen Mittel zur Erzeugung eines dünnen Flüssigkeitsfilms auf der Wärmetauscherfläche sowie Mittel zur Abfuhr der heißen Carotinoid-Lösung von der beheizten Wärmetauscheroberfläche.

Als Dünnschichtapparaturen sind beispielsweise, je nach Art der Filmbildung, Fallfilmverdampfer, einschließlich Drallstromrohr-Fallfilmverdampfer und Wendelrohrfallfilmverdampfer, Blasrohrverdampfer, Rotorverdampfer, einschließlich Luwa-Filmtruder und Sambay-Verdampfer, sowie Zentrifugalverdampfer zu nennen, wobei die vorgenannten Vorrichtungen zur Durchführung des erfindungsgemäßen Verfahrens modifiziert sein können, z. B. durch Weglassen von Mitteln zur Abtrennung von Brüden.

Gemäß einer ersten Ausführungsform Dünnschichtapparatur erfolgt das Erhitzen des Ölstroms, indem man den Ölstrom in einer Dünnschichtapparatur, die eine oder mehrere, vertikal angeordnete Wärmetauscherflächen aufweist, erhitzt. Hierzu wird der Ölstrom im oberen Bereich der vertikal angeordneten Wärmetauscherflächen in einer Weise aufgebracht, dass sich ein dünner Flüssigkeitsfilm der Suspension auf der Wärmetauscherfläche ausbildet. Das Aufbringen kann beispielsweise mit Düsen oder vorzugsweise mittels Wischelementen, im Folgenden auch als Wischer bezeichnet, erfolgen. Der Einsatz von Wischern ist bevorzugt, da durch den Wischvorgang in dem aufgebrachten Flüssigkeitsfilm Turbulenzen erzeugt werden, die einen besonders raschen und gleichmäßigen Wärmeeintrag in den Flüssigkeitsfilm erlauben.

Der so erzeugte Flüssigkeitsfilm läuft aufgrund der Schwerkraft entlang der vertikal angeordneten Wärmetauscherfläche ab und erhitzt sich dabei aufgrund des Kontakts mit der erhitzten Wärmetauscherfläche. Hierbei geht das im Öl suspendierte Carotinoid in Lösung. Am unteren Ende der Wärmetauscherfläche wird die heiße Lösung abgeleitet und der Abschreckung zugeführt.

In einer bevorzugten Ausgestaltung dieser Ausführungsform sind die Wärmetauscherflächen als von außen beheizte, vertikal angeordnete Rohre ausgestaltet, so dass die Suspension über die Innenwände des bzw. der Rohre geführt und dort erhitzt wird.

Eine typische Ausgestaltung sind Fallfilmverdampfer, einschließlich Drallstromrohr-Fallfilmverdampfer und Wendelrohrfallfilmverdampfer. Hierbei handelt es sich um Vorrichtungen, die ein vertikal angeordnetes Rohrbündel aufweisen, wobei die Rohre von außen beheizt werden. Die zu erhitzende Carotinoid-Suspension wird hierbei mittels Düsen, gegebenenfalls über einen Verteiler am oberen Ende der beheizten Rohre, aufgebracht und läuft als gleichmäßiger Film an den Innenwänden der Rohre ab. In der Regel werden die Flüssigkeitsströme unmittelbar am unteren Ende der Rohre vereinigt und der Abschreckung zugeführt. Alternativ kann man so vorgehen, dass man die Rohre nur im oberen Bereich beheizt und im unteren Bereich kühlt, so dass man auf diese Weise das Erhitzen der Carotinoid-Suspension und das Abschrecken der dabei erhaltenen Carotinoid-Lösung in einer einzigen Apparatur durchführt.

Die Rohre des Rohrbündels können gerade oder wendelförmig mit vertikaler Achse (entsprechend einem Wendelrohr-Fallfilmverdampfer) sein. Der Rohrquerschnitt kann beliebig geformt sein und ist in der Regel oval, wie beispielsweise kreisförmig oder ellipsenförmig, oder kann auch ein Polygon sein. Die Rohrinnenwände können glatt sein oder Vertiefungen, z. B. schraubenförmige eingewalzte Sicken (entsprechend der Anordnung in einem Drallstromrohr-Fallfilmverdampfer), aufweisen.

In einer besonders bevorzugten Ausgestaltung der Erfindung weist die Dünnschichtapparatur ein vertikales, von außen beheiztes Rohr mit kreisförmigem Rohrquerschnitt auf, in dessen Inneren sich ein vertikalachsiger Rotor mit Wischelementen/Wischern befindet, der sich zumindest über den beheizten Bereich des Rohres erstreckt. Die Wischer können dabei starr mit dem Rotor verbunden sein (Starrflügelrotor) oder beweglich montiert sein.

Die zu erhitzende Suspension wird im oberen Bereich des Rohres eingespeist und, gegebenenfalls über einen Verteilerring, gleichmäßig auf den Umfang des Rohres verteilt. Die Suspension wird dabei von den Wischelementen des Rotors erfasst und gleichmäßig als dünner Film auf die Rohrinnenwand verteilt. Hierbei bilden sich im Bereich des Spalts zwischen Rohrinnenwand und Wischelement turbulente Zonen im Flüssigkeitsfilm aus, die zu einem raschen und gleichmäßigen Wärmeeintrag in den Flüssigkeitsfilm führen. Der Flüssigkeitsfilm läuft an den Innenwänden der Rohrs ab und wird unmittelbar am unteren Ende des beheizten Rohrs der Abschreckung zugeführt. Bevorzugt wird man so vorgehen, dass man die Rohre nur im oberen Bereich erhitzt und im unteren Bereich kühlt, so dass man auf diese Weise das Erhitzen der Carotinoid-Suspension und das Abschrecken der dabei erhaltenen Carotinoid-Lösung in einer einzigen Apparatur durchführt.

In einer anderen bevorzugten Ausgestaltung dieser Ausführungsform sind die Wärmetauscherflächen als von außen beheizte Wendelrohre mit vorzugsweise vertikaler Anordnung der Wendelachse ausgestaltet. Derartige Apparate sind beispielsweise beschrieben in Chemie-Ing. Tech., 1970, Nr. 6, 349 ff. Hierzu gibt man die zu erhitzende Carotinoid-Suspension am oberen Ende des Wendelrohrs auf, in einer Weise, dass sich ein dünner Flüssigkeitsfilm der Suspension auf der Innenfläche der Wendelrohre ausbildet. Dies kann beispielsweise in analoger Weise zu der oben beschriebenen Fallfilmverdampferanordnung erfolgen. Vorzugsweise wird man parallel zum Strom der Suspension ein inertes Gas in das Wendelrohr einspeisen. Beispiele für inerte Gase sind insbesondere Stickstoff, Luft-Stickstoffgemische mit einem Sauerstoffanteil unterhalb 5 Vol.-%, sowie Wasserdampf. Die Menge des inerten Gases wird dabei so gewählt, dass sich im Wendelrohr eine wellige Filmströmung einstellt, so dass ein intensiver Wärme- und Stoffaustausch gewährleistet ist. Die aufzugebende Menge an Gas kann vom Fachmann durch Versuche oder entsprechende Rechnungen ermittelt werden. Gleichzeitig bewirkt das eingespeiste Gas einen Transport der Flüssigkeit in Strömungsrichtung des eingespeisten inerten Gases. Die den beheizten Teil der Wendelrohre verlassende heiße Carotinoid-Lösung wird unmittelbar der Abschreckung zugeführt. Beispielsweise kann man so vorgehen, dass man die Rohre nur einem ersten, der Einspeisung zugewandten Bereich erhitzt und die Rohre in dem der Einspeisung abgewandten Bereich kühlt, so dass man auf diese Weise das Erhitzen des Ölstroms und das Abschrecken der dabei erhaltenen Carotinoid-Lösung in einer einzigen Apparatur durchführt.

Die Art der Beheizung der Wärmetauscherflächen ist von untergeordneter Bedeutung und kann mittels Heißdampf, Sole, einer Heizflüssigkeit oder mittels Induktionswärme erfolgen.

Die Art der Wärmetauscheranordnung, die Temperatur der Wärmetauscheroberfläche und die mittlere Kontaktzeit der Carotinoid-Suspension mit der Wärmetauscherfläche werden so gewählt, dass man vor dem Abschrecken in Schritt iii. eine heiße Carotinoid-Lösung erhält. Diese Parameter richten sich in an sich bekannter Weise nach der Art des Carotinoids, der gewünschten Konzentration des Carotinoids in der heißen Lösung und der Art des für Nahrungsmittel geeigneten Öls. Die erforderlichen Parameter kann der Fachmann durch übliche Routineverfahren ermitteln.

Vorzugsweise werden diese Parameter so gewählt, dass die Temperatur der erhaltenen heißen Carotinoid-Lösung im Bereich von 50 bis 200 °C, insbesondere im Bereich von 100 bis 180 °C und speziell im Bereich von 120 bis 160 °C liegt.

Vorzugsweise erfolgt das Erhitzen des Ölstroms möglichst rasch, so dass die mittlere Kontaktzeit der Suspension bzw. der heißen Carotinoid-Lösung mit der beheizten Wärmetauscherfläche, gerechnet von Beginn des Kontakts der Suspension mit der beheizten Wärmetauscherfläche bis zum Abschrecken, möglichst kurz ist und eine Dauer von 30 Sekunden, insbesondere 20 Sekunden, bevorzugt 10 Sekunden und speziell 5 Sekunden nicht überschreitet. Typischerweise wird die mittlere Verweilzeit der heißen Carotinoid-Lösung bis zum Abschrecken 0,1 bis 30 Sekunden, insbesondere 0,1 bis 20 Sekunden, bevorzugt 0,1 bis 10 Sekunden und speziell 0,5 bis 5 Sekunden betragen.

Die Oberfläche der beheizten Wärmetauscherfläche wird in der Regel eine Temperatur aufweisen, die oberhalb der Temperatur liegt, auf welche die Suspension erhitzt werden soll. Typischerweise liegt die Oberflächentemperatur wenigstens 10 K, insbesondere wenigstens 20 K, z. B. 10 bis 100 K, und insbesondere 20 bis 50 K oberhalb der Temperatur, auf welche die Carotinoid-Suspension zum Lösen des Carotinoids erhitzt werden soll. Typischerweise liegt die Oberflächentemperatur der beheizten Wärmetauscherfläche wenigstens 40 K und insbesondere wenigstens 50 K, z. B. 40 bis 250 K, und insbesondere 50 bis 200 K oberhalb derjenigen Temperatur, welche die Carotinoid-Suspension unmittelbar vor dem Erhitzen aufweist.

Die Konzentration des Carotinoids in der heißen Carotinoid-Lösung sollte vorzugsweise im Bereich von 0,5 bis 30 g/kg und insbesondere im Bereich von 1 bis 20 g/kg liegen. Die Konzentration in der heißen Carotinoid-Lösung kann über die Konzentration an Carotinoid in der Suspension und die Menge des gegebenenfalls zugeführten, für Nahrungsmittel geeigneten Öls eingestellt werden. Hierbei ist es möglich, vor oder während des Erhitzens die Suspension mit einem für Nahrungsmittel geeigneten Öl zu verdünnen, um die gewünschte Konzentration einzustellen oder eine entsprechend konzentrierte bzw. verdünnte Carotinoid-Suspension herzustellen.

Gegebenenfalls ist es von Vorteil, die Carotinoid-Suspension vor dem eigentlichen Erhitzen auf eine Temperatur vorzuwärmen, bei der ein Lösen der Carotinoid-Partikel in der Ölphase der Suspension noch nicht stattfindet, z. B. auf Temperaturen bis 80 °C, insbesondere auf Temperaturen im Bereich von 40 bis 80 °C. Die Maximaltemperatur, bei der die Lösungsgeschwindigkeit der suspendierten Carotinoid-Partikel in der Ölphase ausreichend langsam ist, so dass ein Lösen nicht stattfindet, hängt naturgemäß vom eingesetzten Carotinoid, von der Größe der Carotinoid-Partikel sowie vom verwendeten Öl ab und kann vom Fachmann durch Routineexperimente ermittelt werden.

In Schritt iii. des erfindungsgemäßen Verfahrens wird die in Schritt ii. erhaltene, heiße Carotinoid-Lösung abgeschreckt, d. h. möglichst rasch abgekühlt, um eine Zersetzung und/oder Isomerisierung des Carotinoids in der heißen Carotinoid-Lösung zu minimieren.

Das Abschrecken kann grundsätzlich in beliebiger Weise erfolgen, sofern ein rasches Abkühlen der heißen Carotinoid-Lösung gewährleistet ist. Bevorzugt wird das Abschrecken so ausgestaltet, dass die heiße Carotinoid-Lösung innerhalb von maximal 30 sec., vorzugsweise innerhalb von maximal 20 sec. und insbesondere innerhalb von maximal 10 sec. auf eine Temperatur von höchstens 60 °C abgekühlt wird.

Gemäß einer ersten Ausführungsform erfolgt das Abschrecken durch Vermischen der heißen Carotinoid-Lösung mit einem kühleren Ölstrom. Das Vermischen der heißen Carotinoid-Lösung mit dem kühleren Ölstrom kann in beliebiger Weise erfolgen, z. B. durch Eintragen der heißen Carotinoid-Lösung in einen großen Überschuss eines kühleren, für Nahrungsmittel geeigneten Öls, das gegebenenfalls Carotinoid gelöst enthält, oder vorzugsweise durch Einspeisen eines Volumenstroms eines kühleren Ölstroms in den Strom der heißen Carotinoid-Lösung.

Als kühlerer Ölstrom können ein für Nahrungsmittel geeignetes Öl oder vorzugsweise eine kühlere Lösung des Carotinoids in einem für Nahrungsmittel geeigneten Öl verwendet werden.

Die Temperatur des kühleren Ölstroms wird in der Regel wenigstens 50 K, häufig wenigstens 80 K und insbesondere wenigstens 100 K unterhalb der Temperatur liegen, welche die heiße Carotinoid-Lösung nach Beendigung des Erhitzens, d. h. nach Verlassen der Heizzone, aufweist.

Typischerweise wird man die Menge und Temperatur des zum Abschrecken verwendeten kühleren Ölstroms so wählen, dass die Temperatur der durch Vermischen erhaltenen Carotinoid-Lösung nicht mehr als 60 °C, insbesondere nicht mehr als 50 °C und speziell nicht mehr als 40 °C beträgt und beispielsweise im Bereich von 10 bis 60 °C, insbesondere im Bereich von 15 bis 50 °C und speziell im Bereich von 20 bis 40 °C liegt. Typischerweise weist der zum Abschrecken verwendete kühlere Ölstrom eine Temperatur im Bereich von 10 bis 60 °C und insbesondere im Bereich von 20 bis 50 °C und speziell im Bereich von 20 bis 30 °C auf.

Das Volumenstromverhältnis bzw. das Verhältnis der Flussraten von heißer Carotinoid-Lösung zu dem kühleren Ölstrom liegt typischerweise im Bereich von 1 : 500 bis 1 : 1 und speziell im Bereich von 1 : 300 bis 1 : 2.

Gemäß einer bevorzugten Ausgestaltung dieser Ausführungsform erfolgt das Vermischen des kühleren Ölstroms mit der heißen Carotinoid-Lösung in einer Mischpumpe. Die Verwendung von Mischpumpen führt zu einer besonders raschen Abkühlung der heißen Carotinoid-Lösung, und ein Auskristallisieren des Carotinoids wird vollständig vermieden. Hierbei ist zu beachten, dass bereits geringe Mengen an nicht gelöstem Carotinoid in der Produktlösung stören und sich auch nur äußerst langsam, wenn überhaupt, wieder lösen. Als Mischpumpen sind grundsätzlich alle für die Förderung von Flüssigkeit geeignete Pumpen zu verstehen, die saugseitig zwei Anschlüsse für die zu mischenden Flüssigkeiten aufweisen. Beispiele für geeignete Mischpumpen sind insbesondere Pumpen mit rotierenden Fördermitteln wie Kreiselradpumpen, insbesondere als Peripheralpumpe bzw. Peripheralradpumpen ausgestaltete Kreiselpumpen, weiterhin Rotationspumpen wie Zahnrad- und Kreiskolbenpumpen, sowie Rotor-Stator-Mischer. In einer bevorzugten Ausführungsform der Erfindung erfolgt das Vermischen der heißen Carotinoid-Suspension mit dem kühleren Ölstrom unter Verwendung einer Peripheralpumpe, die auch als Reaktionsmischpumpe bezeichnet wird und beispielsweise unter der Bezeichnung "Reaktionsmischpumpe" kommerziell erhältlich ist, z. B. von der Fa. K-Engineering Mischtechnik und Maschinenbau, Westoverledingen, DE.

Bei dem zum Abschrecken verwendeten kühleren Ölstrom bzw. kühleren Öl handelt es sich beispielsweise um einen Strom eines für Nahrungsmittel geeigneten Öls, der gleich dem zur Herstellung der Suspension bzw. dem beim Erhitzen zugeführten Öl oder davon verschieden ist, oder um eine Lösung des Carotinoids in einem für Nahrungsmittel geeigneten Öl. Die Art des im kühleren Ölstrom enthaltenen Öls ist für die Erfindung von untergeordneter Bedeutung, so dass es grundsätzlich aus allen der vorgenannten, für Nahrungsmittel geeigneten Ölen ausgewählt sein kann. Vorzugsweise ist es unter Sojabohnenöl, Palmöl, Palmkernöl, Sonnenblumenöl, Distelöl, Maiskeimöl, Olivenöl, Leinsamenöl, Rapsöl, Reisöl, Kokosöl, Erdnussöl, PUFA-Öle, MCT-Ölen, weiterhin Fischölen, Fischölblends sowie Mischungen dieser Öle ausgewählt, insbesondere unter einem Öl oder einer Ölmischung aus der folgenden Gruppe: Maisöl, Sonnenblumenöl, Sojabohnenöl, Fischöl, Leinsamenöl, Rapsöl, Reisöl, Kokosöl und Erdnussöl.

Gemäß einer bevorzugten Ausführungsform der Erfindung handelt es sich bei dem kühleren Ölstrom um eine Lösung des Carotinoids in einem für Nahrungsmittel geeigneten Öl. Durch Verwendung einer Carotinoid-Lösung zum Abschrecken bzw. Verdünnen der heißen Carotinoid-Lösung kann eine übermäßige Verdünnung vermieden werden. Als Folge sind geringere Carotinoid-Konzentrationen in der heißen Carotinoid-Lösung notwendig, um die gewünschte Endkonzentration einzustellen. Dies hat zur Folge, dass die zum Auflösen des Carotinoids erforderlichen Temperaturen verringert werden können, so dass geringere Abbaureaktionen des Carotinoids in der heißen Lösung auftreten. Ein weiterer Vorteil besteht darin, dass die Gefahr eines vorzeitigen Auskristallisierens des Carotinoids aus dem Öl verringert wird. Sofern zum Erhitzen der Carotinoid-Suspension ein heißes Öl verwendet wird, kann die Temperatur des heißen Öls verringert werden. Dies hat den Vorteil, dass Zersetzungsreaktionen des Öls und eine damit einhergehende Belagsbildung an Apparateteilen (sog. Fouling), die insbesondere bei nicht-raffinierten Ölen eine große Rolle spielen, verringert werden können.

Die Konzentration an Carotinoid in der kühleren Carotinoid-Lösung liegt typischerweise im Bereich von 0,005 bis 2 g/kg und insbesondere im Bereich von 0,01 bis 1,5 g/kg.

Das Vermischen der heißen Carotinoid-Lösung mit dem kühleren Ölstrom kann in beliebiger Weise erfolgen, z. B. durch Eintragen der heißen Carotinoid-Lösung in einen großen Überschuss eines kühleren, für Nahrungsmittel geeigneten Öls oder einer kühleren Lösung eines Carotinoids in einem für Nahrungsmittel geeigneten Öl oder vorzugsweise durch Einspeisen eines Volumenstroms des kühleren Öls bzw. der kühleren Carotinoid-Lösung in den Strom der heißen Carotinoid-Lösung.

In einer zweiten, besonders bevorzugten Ausführungsform der Erfindung erfolgt das Abschrecken in einer gekühlten Dünnschichtapparatur, d. h. das Abschrecken erfolgt durch Überleiten der heißen Carotinoid-Lösung über eine gekühlte Wärmetauscherfläche.

Zu diesem Zweck kommen grundsätzlich die zuvor genannten Dünnschichtapparaturen in Betracht, wobei solche Dünnschichtapparaturen bevorzugt sind, die als Wärmetauscher ein oder mehrere von außen gekühlte Rohre, z. B. ein Rohrbündel außengekühlter Rohre oder insbesondere ein von außen gekühltes Rohr mit kreisförmigem Rohrquerschnitt, in dessen Inneren sich ein vertikalachsiger Rotor mit Wischelementen/Wischern befindet, der sich zumindest über den gekühlten Bereich des Rohres erstreckt, aufweisen. Die Wischer können dabei starr mit dem Rotor verbunden sein (Starrflügelrotor) oder beweglich montiert sein.

Hierbei hat es sich als besonders vorteilhaft erwiesen, wenn man das Erhitzen und das Abschrecken in einer einzigen Dünnschichtapparatur durchführt, die sowohl beheizte als auch gekühlte Wärmetauscherflächen aufweist. Hierbei wird die Carotinoid-Suspension zunächst über eine beheizte Wärmetauscherfläche und anschließend über eine gekühlte Wärmetauscherfläche geleitet. Beispielsweise kann man bei vertikaler Anordnung der Wärmetauscherflächen die oberen Bereiche der Wärmetauscherflächen beheizen und die unteren Bereiche kühlen, wie oben am Beispiel eines Rohrbündelwärmetauschers sowie am Beispiel eines Wärmetauscherrohrs mit rotierenden Wischelementen erläutert.

Die Art der Wärmetauscheranordnung, die Temperatur der gekühlten Wärmetauscheroberfläche und die mittlere Kontaktzeit der heißen Carotinoid-Lösung mit der gekühlten Wärmetauscherfläche werden so gewählt, dass man nach dem Abschrecken in Schritt iii. eine Carotinoid-Lösung erhält, deren Temperatur vorzugsweise nicht mehr als 60 °C, insbesondere nicht mehr als 50 °C und speziell nicht mehr als 40 °C beträgt und beispielsweise im Bereich von 10 bis 60 °C, insbesondere im Bereich von 15 bis 50 °C und speziell im Bereich von 20 bis 40 °C liegt. Die erforderlichen Parameter kann der Fachmann durch übliche Routineverfahren ermitteln.

Die Oberfläche der gekühlten Wärmetauscherfläche wird in der Regel eine Temperatur aufweisen, die eine rasche Abkühlung der heißen Carotinoid-Lösung auf die gewünschte Temperatur gewährleistet. Typischerweise liegt die Oberflächentemperatur zumindest teilweise unterhalb der Temperatur, auf welche die heiße Carotinoid-Lösung gekühlt werden soll und liegt an den wärmsten Stellen in der Regel wenigstens 50 K, insbesondere wenigstens 100 K unterhalb der Temperatur, welche die heiße Carotinoid-Lösung nach dem Erhitzen aufweist. Typischerweise liegt die Oberflächentemperatur wenigstens 10 K, insbesondere wenigstens 20 K, z. B. 10 bis 100 K, und insbesondere 20 bis 50 K unterhalb der Temperatur, welche die heiße Carotinoid-Lösung aufweist. Es kann von Vorteil sein, wenn die Wärmetauscherfläche ein Temperaturprofil dergestalt aufweist, dass die gekühlte Wärmetauscherfläche, mit der die heiße Carotinoid-Lösung zu Beginn des Abschreckens in Kontakt kommt, eine höhere Temperatur aufweist als die Wärmetauscherfläche, mit der die Carotinoid-Lösung am Ende des Abschreckens in Kontakt steht. Beispielsweise kann diese Temperaturdifferenz 10 bis 30 K betragen.

Gegebenenfalls kann es von Vorteil sein, vor oder während des Abschreckens in der Dünnschichtapparatur einen kühleren Ölstrom zuzuführen, um das Abschrecken zu fördern und/oder die gewünschte Konzentration an Carotinoid in der abgekühlten Lösung einzustellen.

Gegebenenfalls kann das Abschrecken der heißen Carotinoid-Lösung weitere oder andere Maßnahmen zum Abkühlen umfassen. Zu nennen sind beispielsweise Wärmetauscher, Flash-Verdampfung oder sonstige Maßnahmen, welche vorzugsweise dem Vermischen der heißen Carotinoid-Lösung mit dem kühleren Ölstrom nachgeschaltet sind.

Die nach dem Abschrecken erhaltene Carotinoid-Lösung weist, je nach Löslichkeit des Carotinoids in dem verwendeten essbaren Öl, eine Carotinoid-Konzentration im Bereich von 0,01 bis 3 g/kg, häufig im Bereich von 0,02 bis 2 g/kg und insbesondere im Bereich von 0,05 bis 1,5 g/kg auf. Daneben kann die gemischte Carotinoid-Lösung übliche Stabilisatoren wie Antioxidationsmittel und dergleichen in den oben angegebenen Mengen enthalten. Typischerweise beträgt die Menge an Stabilisatoren 0,01 bis 20 g/kg der gemischten Carotinoid-Lösung.

Sofern zum Abschrecken der heißen Carotinoid-Lösung eine kühlere Lösung des Carotinoids verwendet wird, kann diese in beliebiger Weise hergestellt werden, beispielsweise nach den im Stand der Technik beschriebenen Verfahren. Insbesondere handelt es sich bei der kühleren Carotinoid-Lösung um eine Carotinoid-Lösung, die nach dem erfindungsgemäßen Verfahren hergestellt und gegebenenfalls mit einem für Nahrungsmittel geeigneten Öl verdünnt wurde. Eine solche kühlere Carotinoid-Lösung kann man beispielsweise dadurch erhalten, dass man zumindest einen Teilstrom der in Schritt iii. erhaltenen Carotinoid-Lösung oder die Gesamtmenge dieser Lösung in ein für Nahrungsmittel geeignetes Öl oder in eine Lösung des Carotinoids in einem für Nahrungsmittel geeigneten Öl zurückführt.

Gemäß einer weiteren bevorzugten Ausführungsform führt man die Gesamtmenge der abgeschreckten Carotinoid-Lösung, gegebenenfalls nach weiterer Abkühlung dieser Lösung, in ein für Nahrungsmittel geeignetes Öl zurück. Auf diese Weise erhält man eine verdünnte kühlere Lösung des Carotinoids. Beispielsweise kann man so vorgehen, dass man die nach dem Abkühlen erhaltene Carotinoid-Lösung in ein Vorratsgefäß fährt, das ein für Nahrungsmittel geeignetes Öl enthält. Nach einer kurzen Initialphase erreicht man in dieser Lösung eine für das erfindungsgemäße Verfahren geeignete Carotinoid-Konzentration, wie oben angegeben, und kann dann die so erhaltene Lösung zum Abschrecken der heißen Carotinoid-Lösung einsetzen. Im Verlauf der weiteren Durchführung des erfindungsgemäßen Verfahrens steigt die Carotinoid-Konzentration in der Carotinoidlösung im Vorratsgefäß weiter an. Wenn die gewünschte Konzentration erreicht ist, kann man eine Teilmenge oder die Gesamtmenge der Lösung austragen und durch frisches Öl ersetzen. Man kann aber auch kontinuierlich oder portionsweise die Carotinoid-Lösung ausschleusen und nach Maßgabe ihrer Entnahme durch frisches Öl ersetzen.

Gemäß einer weiteren bevorzugten Ausführungsform führt man einen Teilstrom der gemischten Carotinoid-Lösung, gegebenenfalls nach weiterer Abkühlung der gemischten Lösung, in ein für Nahrungsmittel geeignetes Öl zurück. Der zurückgeführte Teilstrom macht in der Regel 50 bis 99,7 Vol-%, insbesondere 60 bis 99,5 Vol.-%, bezogen auf das Gesamtvolumen der Schritt iii. erhaltenen gemischten Carotinoid-Lösung aus. Beispielsweise kann man so vorgehen, dass man den Teilstrom nach Maßgabe der benötigten Menge an kühlerer Lösung des Carotinoids mit frischem, essbarem Öl vermischt und die so erhaltene Lösung des Carotinoids zum Abschrecken verwendet. Man kann auch so vorgehen, dass man den zurückgeführten Teilstrom in ein Vorratsgefäß fährt, das ein für Nahrungsmittel geeignetes Öl enthält, und aus dem Vorratsgefäß die zum Abschrecken der heißen Carotinoid-Lösung benötigte kühlere Lösung des Carotinoids entnimmt. Durch die Rückführung des Teilstroms in das Vorratsgefäß erhält man eine verdünnte Lösung des Carotinoids in dem für Nahrungsmittel geeigneten Öl, deren Konzentration zunächst ansteigt. Wenn die gewünschte Konzentration erreicht ist, d. h. diejenige Konzentration, welche die zum Abschrecken verwendete kühlere Carotinoid-Lösung idealerweise aufweist, wird man in der Regel die Konzentration durch Zufuhr von frischem Öl konstant halten. Bevorzugte Konzentrationen an Carotinoid liegen im Bereich von 0,005 bis 2 g/kg und insbesondere im Bereich von 0,01 bis 1,5 g/kg.

Gegebenenfalls kann der zurückgeführte Teil- oder Gesamtstrom vor dem Vermischen mit dem für Nahrungsmittel geeigneten Öl oder mit der Lösung des Carotinoids in dem für Nahrungsmittel geeigneten Öl gekühlt werden, um einen Temperaturanstieg der Carotinoid-Lösung im Vorratsgefäß zu vermeiden.

Es versteht sich von selbst, dass die zur Durchführung des erfindungsgemäßen Verfahrens verwendete Vorrichtung übliche Maßnahmen zur Kontrolle und Steuerung bzw. Regelung der Volumenströme und Mittel zur Kontrolle und Steuerung bzw. Regelung der Temperaturen aufweist. Ebenso können Mittel zur Kontrolle der Konzentrationen an Carotinoid in der Suspension, in der heißen Lösung, in der kühleren Lösung und im Produktstrom vorgesehen werden.

Durch das erfindungsgemäße Verfahren erhält man stabile Lösungen von Carotinoiden in essbaren Ölen. Diese Lösungen zeichnen sich dadurch aus, dass das in ihnen enthaltene Carotinoid einen hohen Anteil an all-trans-Isomeren aufweist, der in der Regel oberhalb 70 %, insbesondere oberhalb 80 % und speziell oberhalb 90 % liegt. Derartige Carotinoid-Lösungen lassen sich nach dem Stand der Technik nur mit sehr viel größerem Aufwand herstellen. Zudem lassen sich Öl-Lösungen von Carotinoiden mit derartig hohen Anteilen an all-trans-Isomeren mit dem erfindungsgemäßen Verfahren in technischem Maßstab sehr viel zuverlässiger erzielen als mit den Verfahren des Standes der Technik.

Die nach dem erfindungsgemäßen Verfahren erhältlichen Carotinoid-Lösungen sind lagerstabil und können vor ihrer weiteren Verwendung über längere Zeiträume aufbewahrt werden, ohne dass es in nennenswertem Umfang zu einem Aktivitätsverlust, z. B. durch Isomerisierung und/oder oxidativen Abbau, kommt. Insbesondere zeichnen sie sich durch einen hohen Anteil an all-trans-Isomeren des eingesetzten Carotinoids aus und durch einen vergleichsweise geringen Anteil an Carotinoid-Abbauprodukten.

Das erfindungsgemäße Verfahren kann direkt in die Prozesse zur Weiterverarbeitung der Carotinoid-Lösungen integriert werden.

Die nach dem erfindungsgemäßen Verfahren erhältlichen Carotinoid-Lösungen eignen sich in vorteilhafter Weise als Zusatz zu Tierfuttermitteln, Nahrungsmitteln für die Humanemährung, Nahrungsergänzungsmitteln, pharmazeutischen Mitteln oder kosmetischen Mitteln. Bevorzugt lassen sich die öligen Zubereitungen bzw. Lösungen als Futtermittelzusatz in der Tierernährung einsetzen, beispielsweise bei der Futtermittelherstellung durch Einmischen in einen Futtermittelteig vor oder während der Extrusion oder durch Auftragen bzw. Aufsprühen auf Futtermittelpellets. Die Anwendung als Futtermittelzusatz erfolgt insbesondere durch direktes Aufsprühen der erfindungsgemäßen Formulierungen, beispielsweise als so genannte "post-pelleting liquid application". Vorzugsweise belädt man die Futtermittelpellets mit den Formulierungen unter vermindertem Druck.

Dementsprechend betrifft die vorliegende Erfindung auch die Herstellung von Futtermitteln, wie Tierfuttermittel, Nahrungsmittel für die Humanernährung, Nahrungsergänzungsmittel, sowie die Herstellung pharmazeutischer Mittel oder kosmetischer Mittel unter Verwendung der erfindungsgemäß hergestellten Carotinoid-Lösungen. Diese Mittel enthalten neben den für diese Mittel üblichen Bestandteilen das wenigstens eine essbare Öl und ein Carotinoid, insbesondere Astaxanthin.

Bevorzugte Ausführungsformen betreffen Tierfuttermittel, insbesondere Fischfuttermittel, die das Öl und das Carotinoid, insbesondere Astaxanthin, umfassen. Derartige Mittel enthalten das in der öligen Zubereitung enthaltene Carotinoid typischerweise in einer Menge von 10 bis 100 ppm, bezogen auf das Gesamtgewicht des Mittels, wobei das Carotinoid in der Regel zu mehr als 70 %, insbesondere zu wenigstens 80 % und speziell zu wenigstens 90 % eine all-trans-Konfiguration aufweist.

Typische Bestandteile in Futtermitteln sind Kohlehydrat-Quellen, insbesondere Getreidemehle wie Weizen- oder Maismehl, Sojabohnenmehl, aber auch Zucker und Zuckeralkohole, weiterhin proteinhaltige Bestandteile wie Sojakonzentrat, Fischmehl, Glutene wie Mais- oder Weizengluten, Öle und Fette, z. B. die zuvor genannten essbaren Öle, aber auch andere essbare Fette pflanzlichen oder tierischen Ursprungs, weiterhin Nutrazeutika wie freie Aminosäuren, deren Salze, Vitamine und Spurenelemente, sowie gegebenenfalls Verarbeitungshilfsmittel, z. B. Gleitmittel, Antiblockmittel, inerte Füllstoffe und dergleichen, und gegebenenfalls Konservierungsmittel. Typische Fischfutterzusammensetzungen enthalten z. B. Getreidemehl in einer Menge von beispielsweise 3 bis 20 Gew.-%, Gluten, z. B. in einer Menge von 1 bis 30 Gew.-%, ein oder mehrere Proteinquellen, z. B. Sojakonzentrat und/oder Fischmehl, z. B. in einer Gesamtmenge von 10 bis 50 Gew.-%, Fette und/oder Öle in einer Menge von beispielsweise 10 bis 50 Gew.-%, gegebenenfalls ein oder mehrere Vitamine in einer Gesamtmenge von beispielsweise 0,1 bis 2 Gew.-% und gegebenenfalls Aminosäuren in einer Menge von beispielsweise 0,1 bis 5 Gew.-%, jeweils bezogen auf die Gesamtmenge der Futtermittelbestandteile.

Eine spezielle Ausführungsform dieser Mittel betrifft Futtermittelpellets, speziell Futtermittelpellets für Fischfutter, die mit der erfindungsgemäß erhältlichen Lösung des Carotinoids, insbesondere mit einer Lösung des Astaxanthins, beladen sind. Derartige Pellets enthaltend das in der Lösung enthaltene Carotinoid, speziell Astaxanthin, typischerweise in einer Menge von 10 bis 100 ppm, bezogen auf das Gesamtgewicht des Futtermittels. Die Herstellung solcher Pellets erfolgt in der Regel durch Besprühen konventioneller Pellets mit einer erfindungsgemäßen öligen Zusammensetzung, vorzugsweise unter vermindertem Druck, wobei das Besprühen kontinuierlich oder vorzugsweise diskontinuierlich erfolgen kann. Beispielsweise kann man die konventionellen Pellets in einem geeigneten Gefäß vorlegen, das Gefäß evakuieren und dann Öl unter Durchmischen der Pellets aufsprühen und anschließend belüften. Auf diese Weise erreicht man ein gleichmäßiges Eindringen der erfindungsgemäßen öligen Zusammensetzung in die Pellets. Gegebenfalls kann man erneut Vakuum anlegen und erneut eine erfindungsgemäße ölige Zusammensetzung oder ein essbares Öl in der zuvor beschriebenen Weise aufsprühen. Auf diese Weise erhält man Pellets, die im Kern das Öl enthalten.

Das erfindungsgemäße Verfahren wird im Folgenden anhand von Figuren und Beispielen näher erläutert.

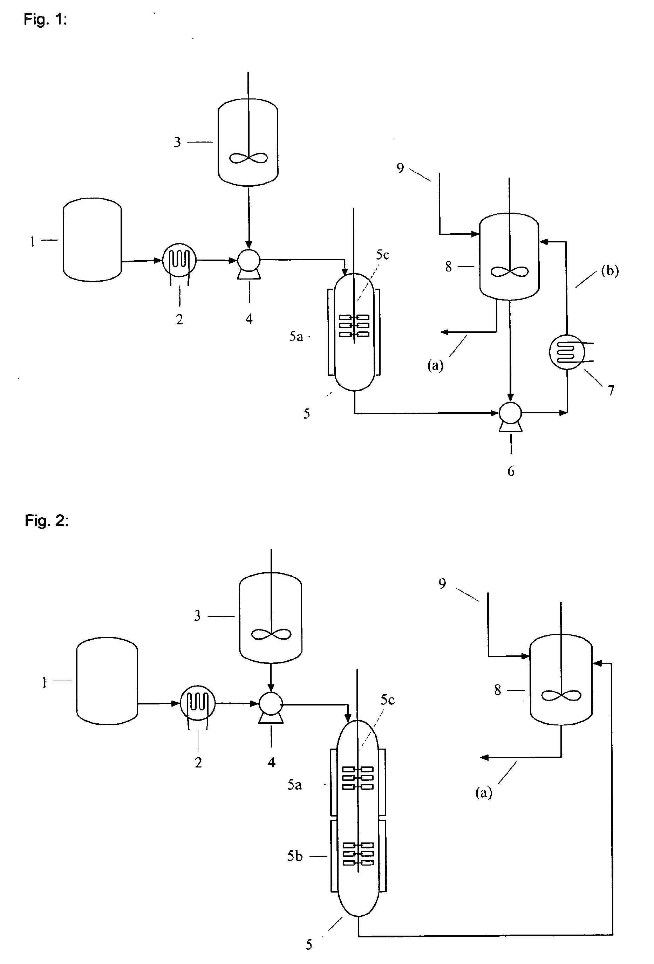

Die Bezugszeichen in den Figuren 1 und 2 haben folgende Bedeutungen:

| | |
|---|---|
| (1) | Vorlagenbehälter für essbares Öl |
| (2) | Wärmetauscher |
| (3) | Vorlagenbehälter für Carotinoid-Suspension |
| (4) | Mischpumpe |
| (5) | Dünnschichtapparatur |
| (5a) | Wärmetauscherrohr beheizt |
| (5b) | Wärmetauscherrohr gekühlt |
| (5c) | rotierende Wischerelemente |
| (6) | Mischpumpe |
| (7) | Wärmetauscher |
| (8) | Vorlagenbehälter für kühlere Carotinoidlösung |
| (9) | Zuleitung für frisches Öl |
| (a) | Produktentnahme |
| (b) | zurückgeführter (Teil-)Strom |

Figur 1 zeigt schematisch eine erste bevorzugte Ausführungsform der Erfindung, bei der das Erhitzen in einem vertikal angeordneten Wärmetauscherrohr mit rotierenden Wischern durchgeführt wird und zum Abschrecken die heiße Carotinoid-Lösung mit einer kühleren Carotinoid-Lösung in einer Mischpumpe vermischt wird.

Gemäß dem in Figur 1 dargestellten Verfahren wird essbares Öl aus dem Vorlagenbehälter (1) in den Wärmetauscher (2) geleitet und dort auf eine Temperatur von 40 bis 80 °C erwärmt. Das warme Öl wird in der Mischpumpe (4) mit der Carotinoid-Suspension aus dem Vorlagenbehälter (3), welcher Mittel zum Bewegen der Suspension wie Rührer, etc. aufweist, vermischt. Das Volumenverhältnis von warmem Öl zu Suspension wird vorzugsweise im Bereich von 1000 : 1 bis 1 : 2 und insbesondere im Bereich von 500 : 1 bis 1 : 1 liegen. Die Carotinoid-Konzentration in der verdünnten Suspension liegt vorzugsweise im Bereich von 1 bis 10 g/kg. Die die Mischpumpe (4) verlassende, verdünnte Carotinoid-Suspension wird in den oberen Bereich der Dünnschichtapparatur (5) geleitet, wobei die rotierenden Wischelemente (5c) die Suspension auf die Innenwand des beheizten Wärmetauscherrohrs (5a) schleudern und sich ein dünner Flüssigkeitsfilm ausbildet, der auf der Innenwand des beheizten Wärmetauscherrohrs (5a) nach unten abläuft, wobei es zu einer vollständigen Lösung des Carotinoids in dem essbaren Öl kommt. Auf diese Weise sammelt sich im unteren Bereich der Dünnschichtapparatur eine heiße Carotinoid-Lösung an. Die Carotinoid-Konzentration in der heißen Lösung liegt vorzugsweise im Bereich von 1 bis 10 g/kg. Die Temperatur der heißen Carotinoid-Lösung im unteren Bereich der Dünnschichtapparatur liegt vorzugsweise im Bereich von 100 bis 180 °C und insbesondere im Bereich von 120 bis 160 °C. Die heiße Carotinoid-Lösung wird in die Mischpumpe (6) geleitet und mit der kühleren Carotinoid-Lösung zum Produktstrom (b) vermischt. Die Carotinoid-Konzentration in der kühleren Lösung liegt vorzugsweise im Bereich von 0,01 bis 2 g/kg. Die Temperatur der kühleren Carotinoid-Lösung nach Verlassen der Mischpumpe (6) liegt vorzugsweise im Bereich von 20 bis 50 °C und insbesondere im Bereich von 20 bis 30 °C. Das Volumenverhältnis von heißer Lösung zu kühlerer Lösung liegt vorzugsweise im Bereich von 1 : 2 bis 1 : 300.

Der Produktstrom (b) wird, gegebenenfalls nach Passage durch den optionalen Wärmetauscher (7), in den Vorlagenbehälter (8) überführt. Gegebenenfalls wird dem Vorlagenbehälter (8) über die Zuleitung (9) frisches Öl zugeführt, um die Konzentration an Carotinoid in dem Vorlagenbehälter (8) einzustellen. Über die Leitung (a) kann kontinuierlich oder nach Bedarf das Produkt entnommen werden.

Figur 2 zeigt schematisch eine zweite bevorzugte Ausführungsform der Erfindung, bei der das Erhitzen und das Abschrecken in einem vertikal angeordneten Wärmetauscherrohr mit rotierenden Wischern durchgeführt werden.

Gemäß dem in Figur 2 dargestellten Verfahren wird essbares Öl aus dem Vorlagenbehälter (1) in den Wärmetauscher (2) geleitet und dort auf eine Temperatur von 40 bis 80 °C erwärmt. Das warme Öl wird in der Mischpumpe (4) mit der Carotinoid-Suspension aus dem Vorlagenbehälter (3), welcher Mittel zum Bewegen der Suspension wie Rührer, etc. aufweist, vermischt. Das Volumenverhältnis von warmem Öl zu Suspension wird vorzugsweise im Bereich von 1000 : 1 bis 1 : 2 und insbesondere im Bereich von 500 : 1 bis 1 : 1 liegen. Die Carotinoid-Konzentration in der verdünnten Suspension liegt vorzugsweise im Bereich von 1 bis 10 g/kg. Die die Mischpumpe (4) verlassende, verdünnte Carotinoid-Suspension wird in den oberen beheizten Bereich (5a) der Dünnschichtapparatur (5) geleitet, wobei die rotierenden Wischelemente (5c) die Suspension auf die Innenwand des beheizten Wärmetauscherrohrs (5a) verteilen und sich ein dünner Flüssigkeitsfilm ausbildet, der zunächst auf der Innenwand des beheizten Wärmetauscherrohrs (5a) nach unten abläuft, wobei es zu einer vollständigen Lösung des Carotinoids in dem essbaren Öl kommt. Die Carotinoid-Konzentration in der heißen Lösung liegt vorzugsweise im Bereich von 0,01 bis 2 g/kg. Die Temperatur der heißen Carotinoid-Lösung im unteren Bereich der Dünnschichtapparatur liegt vorzugsweise im Bereich von 100 bis 180 °C und insbesondere im Bereich von 120 bis 160 °C. Die heiße Carotinoid-Lösung läuft weiter nach unten, wo sie mit der Innenwand des gekühlten Wärmetauscherrohrs (5b) in Kontakt kommt. Hierbei wird sie auf eine Temperatur vorzugsweise im Bereich von 20 bis 50 °C und insbesondere im Bereich von 20 bis 30 °C gekühlt.

Der Produktstrom (b) wird in den Vorlagenbehälter (8) überführt. Über die Leitung (a) kann kontinuierlich oder nach Bedarf das Produkt entnommen werden.

## Patentansprüche

1. Verfahren zur Herstellung von Carotinoid-Lösungen in einem für Nahrungsmittel geeigneten Öl, umfassend:
i. Bereitstellung einer Suspension des Carotinoids in einem für Nahrungsmittel geeigneten Öl,
ii. kontinuierliches Erhitzen eines Stroms der Carotinoid-Suspension, wobei man eine heiße Carotinoid-Lösung erhält, und
iii. kontinuierliches Abschrecken der heißen Carotinoid-Lösung,
wobei man zum kontinuierlichen Erhitzen die Carotinoid-Suspension als dünnen Flüssigkeitsfilm über eine beheizte Wärmetauscherfläche führt.

2. Verfahren nach Anspruch 1, wobei man das Erhitzen der Carotinoid-Suspension in einer Dünnschichtapparatur mit vertikal angeordneten Wärmetauscherflächen durchführt, wobei die Wärmetauscherfläche als ein oder mehrere, von außen beheizte Rohre ausgestaltet sind.

3. Verfahren nach Anspruch 2, wobei die Carotinoid-Suspension mittels Wischelementen auf die Wärmetauscherfläche aufgebracht wird.

4. Verfahren nach Anspruch 1, wobei man das Erhitzen der Carotinoid-Suspension in von außen beheizten Wendelrohren mit vertikal angeordneter Wendelachse durchführt, wobei man während des Erhitzens der Carotinoid-Suspension in die Wendelrohre parallel zum Strom der Suspension ein inertes Gas einspeist.

5. Verfahren nach einem der vorhergehenden Ansprüche, wobei die Temperatur der heißen Carotinoid-Lösung im Bereich von 50 bis 200 °C liegt.

6. Verfahren nach einem der vorhergehenden Ansprüche, wobei die mittlere Kontaktdauer der Carotinoid-Suspension mit der beheizten Wärmetauscherfläche nicht mehr als 30 sec. beträgt.

7. Verfahren nach einem der vorhergehenden Ansprüche, wobei man zum Abschrecken die heiße Carotinoid-Lösung als dünnen Flüssigkeitsfilm über eine gekühlte Wärmetauscherfläche führt.

8. Verfahren nach Anspruch 7, wobei man das Erhitzen der Carotinoid-Suspension und das Abschrecken der heißen Carotinoid-Lösung in einer Dünnschichtapparatur mit vertikal angeordneten Wärmetauscherflächen, die mit Wischelementen ausgerüstet sind, durchführt, wobei die Wärmetauscherflächen im oberen Bereich der Dünnschichtapparatur beheizt und die Wärmetauscherflächen im unteren Bereich der Dünnschichtapparatur gekühlt werden.

9. Verfahren nach Anspruch 4, wobei man das Abschrecken der heißen Carotinoid-Lösung in von außen gekühlten Wendelrohren durchführt.

10. Verfahren nach Anspruch 4 oder 9, wobei man das Erhitzen der Carotinoid-Suspension und das Abschrecken der heißen Carotinoid-Lösung in Wendelrohren mit vertikal angeordneter Wendelachse durchführt, wobei die Wendelrohre im oberen Bereich beheizt und die Wendelrohre im unteren Bereich gekühlt werden.

11. Verfahren nach einem der Ansprüche 1 bis 6, wobei das Abschrecken der heißen Carotinoid-Lösung das Vermischen der heißen Carotinoid-Lösung mit einem kühleren Strom eines für Nahrungsmittel geeigneten Öls umfasst, wobei das Abschrecken der heißen Carotinoid-Lösung durch Vermischen der heißen Carotinoid-Lösung mit der kühleren Carotinoid-Lösung in einer Mischpumpe erfolgt, wobei die Temperatur des kühleren Stroms eines für Nahrungsmittel geeigneten Öls wenigstens 50 K unterhalb der Temperatur der heißen Carotinoid-Lösung liegt.

12. Verfahren nach einem der vorhergehenden Ansprüche, wobei die Carotinoid-Lösung nach dem Abschrecken eine Temperatur von nicht mehr als 60 °C aufweist.

13. Verfahren nach einem der vorhergehenden Ansprüche, wobei die über die Wärmetauscherfläche geführte Carotinoid-Suspension 0,5 bis 30 g/kg des Carotinoids enthält.

14. Verfahren nach einem der vorhergehenden Ansprüche, wobei man vor oder während des Erhitzens zu der Carotinoid-Suspension ein für Nahrungsmittel geeignetes Öl gibt, wobei die Konzentration des Carotinoids in der nach dem Abschrecken erhaltenen Carotinoid-Lösung im Bereich von 0,01 bis 10 g/kg liegt.

15. Verfahren nach einem der vorhergehenden Ansprüche, wobei das für Nahrungsmittel geeignete Öl ausgewählt ist unter Sojabohnenöl, Palmöl, Palmkernöl, Sonnenblumenöl, PUFA-Ölen, MCT-Ölen, Fischölen, Distelöl, Maisöl, Olivenöl, Leinsamenöl, Rapsöl, Reisöl und Mischungen dieser Öle, und insbesondere ein nicht-raffiniertes, für Nahrungsmittel geeignetes Öl ist.

16. Verfahren nach einem der vorhergehenden Ansprüche, wobei das Carotinoid Astaxanthin ist.

## Claims

1. A method for prepaying carotenoid solutions in an oil suitable for foodstuffs, comprising:
i. provision of a suspension of the carotenoid in an oil suitable for foodstuffs,
ii. continuous heating of a stream of the carotenoid suspension, wherein a hot carotenoid solution is obtained, and
iii. continuous quenching of the hot carotenoid solution,
wherein for the continuous heating, the carotenoid suspension is passed as a thin liquid firm over a heated heat-exchange surface.

2. The method according to clam 1, wherein the carotenoid suspension is heated in a thin-film apparatus having vertically arranged heat-exchange surfaces, wherein the heat-exchange surface is designed as once or more externally heated tubes.

3. The method according to claim 2, wherein the carotenoid suspension is applied to the heat-exchange surface by means of scraper elements.

4. The method according to claim 1, wherein the carotenoid suspension is heated in externally heated helical tubes having a vertically arranged helix axis, wherein, while the carotenoid suspension is being heated, an inert gas is fed info the helical tubes which are parallel to the stream of the suspension.

5. The method according to any one of the preceding claims, wherein the temperature of the hot carotenoid solution is in the range from 50 to 200°C.

6. The method according to any once of the preceding claims, wherein the median contact time between the carotenoid suspension and the heated heat-exchange surface is not greater than 30 sec.

7. The method according to any one of the preceding claims, wherein, for the quenching, the hot carotenoid solution is conducted as a thin liquid film over a cooled heat-exchange surface.

8. The method according to claim 7, wherein the carotenoid suspension is heated and the hot carotenoid solution is quenched in a thin-film apparatus heaving vertically arranged heat-exchange surfaces which are equipped with scraper elements, wherein the heat-exchange surfaces in the upper region of the thin-film apparatus are heated, and the heat-exchange surfaces in the lower region of the thin-film apparatus are cooled.

9. The method according to claim 4, wherein the hot carotenoid solution is quenched in externally cooled helical tubes.

10. The method according to claim 4 or 9, wherein the carotenoid suspension is heated and the hot carotenoid solution is quenched in helical tubes having a vertically arranged helix axis, wherein the helical tubes in the upper region are heated and the helical tubes in the lower region are cooled.

11. The method according to any one of claims 1 to 6, wherein the quenching of the hot carotenoid solution comprises fixing the hot carotenoid solution with a cooler stream of an oil suitable for foodstuffs, wherein the hot carotenoid solution is quenched by mixing the hot carotenoid solution with the cooler carotenoid solution in a fixing pump, wherein the temperature of the cooler stream of an oil suitable for foodstuffs is at least 50 K below the temperature of the hot carotenoid solution.

12. The method according to any one of the preceding claims, wherein the carotenoid solution after the quenching has a temperature of not above 60°C.

13. The method according to any one of the preceding claims, wherein the carotenoid suspension which is conducted over the heat-exchange surface comprises 0.5 to 30 g/kg of the carotenoid.

14. The method according to any one of the preceding claims, wherein an oil suitable for foodstuffs is added to the carotenoid suspension before or during the heating, wherein the concentration of the carotenoid in the carotenoid solution obtained after the quenching is in the range from 0.01 to 10 g/kg.

15. The method according to any one of the preceding claims, wherein the oil suitable for foodstuffs is selected from soybean oil, palm oil, palm kernel oil, sunflower oil, PUFA oils, MCT oils, fish oils, safflower oil, corn oil, olive oil, linseed oil, rapeseed oil, rice oil and mixtures of these oils, and in particular is an unrefined oil suitable for foodstuffs.

16. The method according to any one of the preceding claims, wherein the carotenoid is astaxanthin.

## Revendications

1. Procédé pour la préparation de solutions de caroténoïde dans une huile appropriée pour un aliment, comprenant :
i. la préparation d'une suspension du caroténoïde dans une huile appropriée pour un aliment,
ii. le chauffage continu d'un flux de la suspension de caroténoïde, une solution chaude de caroténoïde étant obtenue, et
iii. le refroidissement brusque contenu de la solution chaude de caroténoïde,
la suspension de caroténoïde étant guindée sous forme d'un film liquide mince sur une surface échangeuse de chaleur chauffée en vue de son chauffage continu.

2. Procédé selon la revendication 1, le chauffage de la suspension de caroténoïde étant réalisé dans un appareil à couche mince présentant des surfaces échangeuse de chaleur disposées verticalement, les surfaces échangeuses de chaleur étant réalisées sous forme d'un ou de plusieurs tubes chauffés de l'extérieur.

3. Procédé selon la revendication 2, la suspension de caroténoïde étant appliquée sur la surface échangeuse de chaleur au moyen d'éléments de type raclette.

4. Procédé selon la revendication 1, le chauffage de la suspension de caroténoïde étant réalisé dans des tubes hélicoïdaux chauffés de l'extérieur, présentant un axe hélicoïdal disposé verticalement, un gaz inerte étant injecté pendant le chauffage de la suspension de caroténoïde dans les tubes hélicoïdaux, parallèlement au flux de la suspension.

5. Procédé selon l'une quelconque des revendications précédente, la température de la solution chaude de caroténoïde étant situés dans la plage de 50 à 200°C.

6. Procédé selon l'une quelconque des revendications précédentes, la durée de contact moyenne de la suspension de caroténoïde avec la surface échangeuse de chaleur chauffée n'étant pas supérieure à 30 secondes.

7. Procédé selon l'une quelconque des revendications précédentes, la solution chaude de caroténoïde étant guidée sous forme d'un film de liquide mince sur une surface échangeuse de chaleur refroidie en vue de son refroidissement brusque.

8. Procédé selon la revendication 7, le chauffage de la suspension de caroténoïde et le refroidissement brusque de la solution chaude de caroténoïde étant réalisés dans un appareil à couche mince présentant des surfaces échangeuses de chaleur disposées verticalement et équipées d'éléments de type raclette, les surfaces échangeuses de chaleur étant chauffées dans la zone supérieure de l'appareil à couche mince et refroidies dans la zone inférieure de l'appareil à couche mince.

9. Procédé selon la revendication 4, le refroidissement brusque de la solution chaude de caroténoïde étant réalisé dans des tubes hélicoïdaux refroidis de l'extérieur.

10. Procédé selon la revendication 4 ou 9, le chauffage de la suspension de caroténoïde et le refroidissement brusque de la solution chaude de caroténoïde étant réalisés dans des tubes hélicoïdaux présentant un axe hélicoïdal disposé verticalement, les tubes hélicoïdaux étant chauffés dans la zone supérieure et refroidis dans la zone inférieure.

11. Procédé selon l'une quelconque des revendications 1 à 6, le refroidissement brusque de la solution chaude de caroténoïde comprenant le mélange de la solution chaude de caroténoïde par un flux plus froid d'une huile appropriée pour un aliment, le refroidissement brusque de la solution chaude de caroténoïde étant réalisé par mélange de la solution chaude de caroténoïde avec la solution plus froide de caroténoïde dans une pompe de mélange, la température du flux plus froid d'une huile appropriée pour un aliment étant située à au moins 50 K sous la température de la solution chaude de caroténoïde.

12. Procédé selon l'une quelconque des revendications précédentes, la solution de caroténoïde présentant une température qui n'est pas supérieure à 60°C après le refroidissement brusque.

13. Procédé selon l'une quelconque des revendications précédentes, la suspension de caroténoïde guidée sur les surfaces échangeuses de chaleur contenant 0,5 à 30 g/kg de caroténoïde.

14. Procédé selon l'une quelconque des revendications précédentes, une huile appropriée pour un aliment étant ajoutée avant ou pendant le chauffage à la suspension de caroténoïde, la concentration en caroténoïde dans la solution de caroténoïde obtenue après le refroidissement brusque étant située dans la plage de 0,01 à 10 g/kg.

15. Procédé selon l'une quelconque des revendications précédentes, l'huile appropriée pour un aliment étant choisie parmi l'huile de germes de soja, l'huile de palme, l'huile de palmiste, l'huile de tournesol, les huiles PUFA (acides gras polyinsaturés), les huiles MCT (triglycérides à chaîne moyenne), les huiles de poisson, l'huile de carthame, l'huile de mais, l'huile d'olive, l'huile de graines de lin, l'huile de colza, l'huile de riz et les mélanges de ces huiles et étant en particulier une huile non raffinée appropriée pour un aliment.

16. Procédé selon l'une quelconque des revendications précédentes, le caroténoïde étant l'astaxanthine.
